# EUROPÄISCHE PATENTSCHRIFT

(11) **EP 2 162 171 B1**
(45) Veröffentlichungstag und Bekanntmachung des Hinweises auf die Patenterteilung: **06.03.2019**
(21) Anmeldenummer: 08773532.0
(22) Anmeldetag: 19.06.2008
(51) Int. Cl.: A61M 5/30, A61M 5/20

(54) **EINWEGINJEKTOR MIT MINDESTENS EINEM ZUGHAKEN**
DISPOSABLE INJECTOR WITH AT LEAST ONE TENSION HOOK
INSTRUMENT D'INJECTION À USAGE UNIQUE COMPRENANT AU MOINS UN CROCHET DE TRACTION

(30) Priorität: 10.07.2007 DE 102007032464
(43) Veröffentlichungstag der Anmeldung: 17.03.2010
(73) Patentinhaber: LTS LOHMANN Therapie-Systeme AG, 56626 Andernach (DE)
(72) Erfinder: MATUSCH, Rudolf, 35041 Marburg (DE)
(74) Vertreter: Siemund, Volker
(86) Internationale Anmeldenummer: PCT/EP2008/004947
(87) Internationale Veröffentlichungsnummer: WO 2009/006984

(56) Entgegenhaltungen:
- WO-A-2006/088513
- WO-A-2007/073839
- US-A- 3 557 784
- US-A- 4 227 528
- US-A- 4 378 015
- US-A1- 2005 020 984
- US-A1- 2006 129 089

## Beschreibung

Die Erfindung betrifft einen Einweginjektor mit einem Gehäuse, in dem oder an dem - jeweils zumindest bereichsweise - mindestens ein mechanischer Federenergiespeicher, mindestens eine - zumindest zeitweise wirkstoffbefüllbare - Zylinder-Kolben-Einheit, mindestens ein Kolbenbetätigungsstempel und mindestens eine Auslöseeinheit angeordnet ist, wobei der Federenergiespeicher mindestens ein vorgespanntes Federelement umfasst und wobei zumindest ein Teil des Kolbenbetätigungsstempels zwischen dem Federenergiespeicher und einem Kolben der Zylinder-Kolben-Einheit positioniert ist, wobei das Gehäuse mindestens einen Zughaken aufweist, wobei der einzelne Zughaken jeweils im Bereich seines freien Endes mindestens eine Abstützfläche hat, wobei an der Abstützfläche der federbelastete Kolbenbetätigungsstempel gegen eine Auslösung des Einweginjektors sperrende Lage anliegt, wobei die zwischen dem Zughaken und dem Kolbenbetätigungsstempel gelegene Kontaktzone ein den Zughaken radial nach außen drängendes Keilgetriebepaar darstellt, wobei die sperrende Lage des Zughakens durch ein in einer Sperrstellung positioniertes Auslöseelement gesichert ist und wobei das Auslöseelement eine Lösestellung hat, die ein radial nach außen gerichtetes Zurückweichen des Zughakens - unter Freigabe des Kolbenbetätigungsstempels - bewirkt.

Ein derartiger Injektor ist aus der US 3,557,784 A bekannt.

Aus der DE 36 44 984 A1 ist u.a. ein derartiger Injektor bekannt. Er hat einen federvorgespannten Kolbenbetätigungsstempel, dessen rückwärtige Stempelstange an ihrem freien Ende elastische Zughaken aufweist. Die Zughaken halten den Kolbenbetätigungsstempel formschlüssig an einer Kante des Injektorgehäuses fest. Sie haben hierzu nur eine geringe Auflagefläche am Gehäuse. Zum Auslösen des Injektors werden die Zughaken von der sie haltenden Kante geschoben. In der Folge schießt der federvorgespannte Kolbenbetätigungsstempel nach vorn, um eine Injektion durchzuführen.

Aus der US 2005/0020984 A1 ist ein hochkomplizierter, vielteiliger Mehrweg-Injektor bekannt. Er hat einen auf Zug belasteten Kolbenbetätigungsstempel, der mittels eines mehrere Finger aufweisenden Rings am Gehäuse abgestützt ist. Die Abstützung erfolgt unter Zwischenschaltung anderer, separater Gehäuseteile.

Der vorliegenden Erfindung liegt daher die Problemstellung zugrunde, einen modular aufgebauten Einweginjektor zu entwickeln, der bei geringer Baugröße nur wenige Bauteile aufweist und bei einfacher Handhabung eine sichere Lagerung und Funktion gewährleistet.

Diese Problemstellung wird mit den Merkmalen des Hauptanspruchs gelöst. Dazu weist das Gehäuse mindestens einen Zughaken auf, der jeweils im Bereich seines freien Endes mindestens eine Abstützfläche hat. An der Abstützfläche liegt der federbelastete Kolbenbetätigungsstempel an, wobei die zwischen dem Zughaken und dem Kolbenbetätigungsstempel gelegene Kontaktzone ein den Zughaken radial nach außen drängendes Keilgetriebepaar darstellt. Die sperrende Lage des Zughakens ist durch ein in einer Sperrstellung positioniertes Auslöseelement gesichert. Das Auslöseelement hat eine Lösestellung, die ein radial nach außen gerichtetes Zurückweichen des Zughakens - unter Freigabe des Kolbenbetätigungsstempels - bewirkt.

Mit der Erfindung wird hier beispielsweise ein nadelfreier Einmalinjektor vorgestellt, dessen Kolbenbetätigungsstempel bei einem Auslösevorgang des Einweginjektors freigegeben wird. Dazu wird zum Vorspannen und Halten des Federenergiespeichers der Kolbenbetätigungsstempel über mindestens einen am Gehäuse angeordneten oder im Gehäuse integrierten Zughaken form- und kraftschlüssig gehalten. Der oder die Zughaken werden von einem Auslöseelement bis zum Gebrauch des Einweginjektors in ihrer Sperrposition gehalten. Zum Auslösen des Injektors werden der oder die Zughaken durch ein Verschieben des Auslöseelements freigegeben, so dass sich der Kolbenbetätigungsstempel - unter der Wirkung des Federenergiespeichers - zumindest annähernd parallel zur Mittellinie des Einweginjektors bewegen kann.

Weitere Einzelheiten der Erfindung ergeben sich aus den Unteransprüchen und den nachfolgenden Beschreibungen von einigen schematisch dargestellten Ausführungsbeispielen.
- Figur 1:: Einweginjektor mit zwei Zughaken und Ettiketsicherung, welcher keinen Teil der Erfindung darstellt;
- Figur 2:: wie Figur 1, jedoch entsichert und betätigt (fiktiver Zustand);
- Figur 3:: wie Figur 2, jedoch nach dem Medikamentenausstoß;
- Figur 4:: Einweginjektor mit zwei Zughaken und Auslösehebelsicherung;
- Figur 5:: Einweginjektor mit zwei in Sperrstellung verformten Zughaken;
- Figur 6:: Querschnitt A-A zu Figur 5 bzw. A'-A' zu Figur 8;
- Figur 7:: Querschnitt B-B zu Figur 5;
- Figur 8:: wie Figur 5, jedoch entsichert;
- Figur 9:: wie Figur 5, jedoch nach dem Medikamentenausstoß;
- Figur 10:: Seitenansicht zu Figur 9, jedoch um 45 Winkelgrade zur Seite geschwenkt;
- Figur 11:: Dimetrische Ansicht zu Figur 8 ohne Auslösekappe.

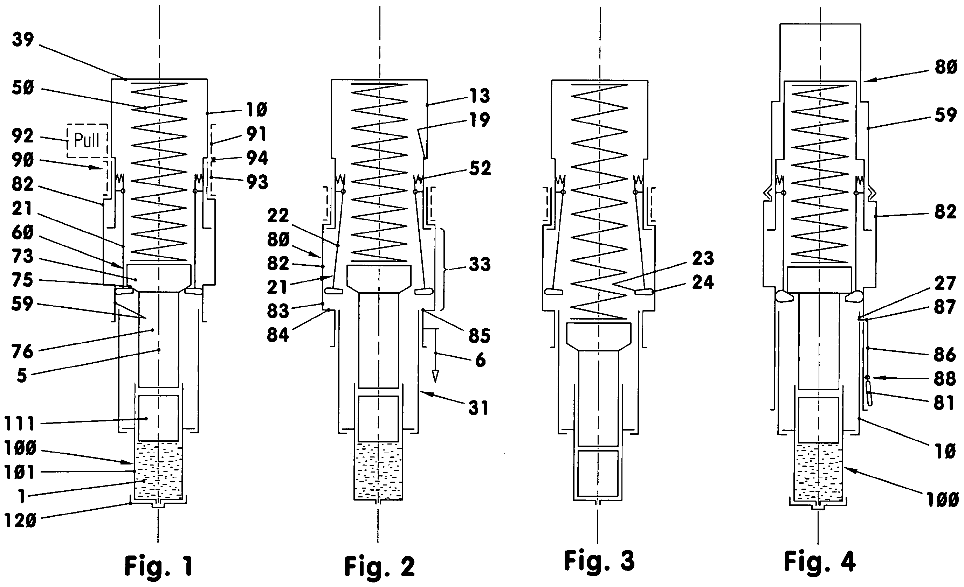

Die Figuren 1 bis 3 zeigen ein vereinfachtes Prinzip eines Einweginjektors mit einem dauergeladenen Federenergiespeicher. Der Einweginjektor besteht aus einem Gehäuse (10), einer z.B. mit einer Injektionslösung befüllten Zylinder-Kolben-Einheit (100), einem Kolbenbetätigungsstempel (60) und einer Schraubendruckfeder (50) als Federenergiespeicher. Zudem sind am Gehäuse (10) ein Auslöseelement (82) und ein Sicherungselement (90) angeordnet.

Das Gehäuse (10) ist ein topfförmiger, unten offener Hohlkörper mit obenliegendem Boden (39). Im mittleren Bereich, dem Mantelbereich (31), hat das Gehäuse (10) z.B. zwei einander gegenüberliegende fensterartige Durchbrüche (33). Im Bereich des jeweils oberen Randes des einzelnen Durchbruchs (33) ist je ein Zughaken (21) gelenkig gelagert. Die Zughaken (21) stützen sich dabei mittels der Federelemente (52) am Gehäuse (10) ab. Die Federelemente (52), z.B. kleine Schraubendruckfedern, liegen oberhalb der Schwenkgelenke und versuchen die unteren, freien Enden der Zughaken radial nach außen zu pressen.

Die beiden Zughaken (21) halten den Kolbenbetätigungsstempel (60) an dessen Stempelteller (73) in seiner vorgespannten Lage. Dazu umgreifen die Zughaken (21) mit ihren Abstützflächen (23) - und/oder - kanten die untere Bundfläche (75) des Stempeltellers (73). Die Größe der jeweiligen Kontaktfläche zwischen einer Abstützfläche (23) und der entsprechenden Stirnfläche (75) liegt im Bereich von 2 bis 20 mm².

Bei den Figuren 1 bis 3 hat die Bundfläche (75) die Form eines Kegelstumpfmantels, dessen Spitzenwinkel ca. 100 bis 130, vorzugsweise 120 Winkelgrade beträgt. Die gedachte Spitze des Kegelstumpfmantels liegt auf der Mittellinie (5) im Bereich des Kolbenschiebers (76). Die Zughaken (21) liegen mit schmalen, fast linienförmigen Abstützflächen (23) an der Bundfläche (75) an. Gerade umgekehrt verhalten sich die Kontaktflächen bei Figur 4. Dort haben die Zughaken (21) großflächige Abstützflächen (23), die jeweils Teil eines Kegelstumpfmantels sind. An diesen Abstützflächen (23) liegt der Stempelteller (73) über seine beispielsweise abgerundete Außenkante auf.

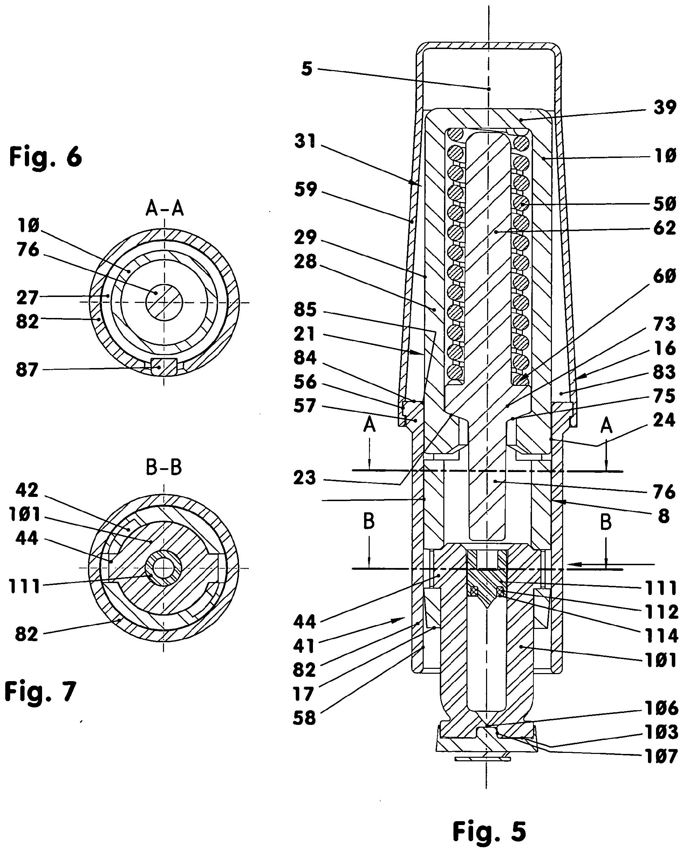

Eine dritte Art der Kontaktflächengestaltung wäre das Aneinanderliegen großflächiger Kontaktflächen, vgl. hierzu Figur 5. Dort berühren sich die Bundfläche (75) und die Abstützfläche (23) vollflächig.

Auf der der Mittellinie (5) abgewandten Seite weist jeder Zughaken (21) eine z.B. gekrümmte Anlagefläche (24) auf.

Im unteren Bereich des Gehäuses (10) befinden sich Halteelemente zur Befestigung der Zylinder-Kolben-Einheit.

Die Zylinder-Kolben-Einheit (100) besteht im Ausführungsbeispiel aus einem, mit einer Injektionslösung (1) befüllten, Zylinder (101), in dem ein Kolben (111) in der hinteren Position sitzt. Oberhalb des Kolbens (111) ist im Gehäuse (10) der Kolbenbetätigungsstempel (60) z.B. so angeordnet, dass er den Kolben zwar nicht berührt, jedoch mit seinem unteren Ende im oberen Bereich des Zylinders (101) seitlich geführt wird.

Nach Figur 1 ist das Gehäuse (10) im mittleren Bereich von dem hülsenartigen, z.B. die Durchbrüche (33) abdeckenden Auslöseelement (82) umgeben. Das Auslöseelement (82) ist auf der radialen Außenfläche (13) des Gehäuses (10) längsverschiebbar gelagert. Es hat im mittleren Bereich eine umlaufende Aufweitung (83). Anstelle dieser Aufweitung (83) können bei einem nichtrotationssymmetrischen Auslöseelement (82) pro Zughaken (21) auch partielle Aufweitungen oder nicht abgedeckte Öffnungen vorhanden sein.

Die Aufweitung (83) ist im Bezug auf das Gehäuse (10) genau so positioniert und dimensioniert, dass sie die beim Auslösevorgang zurückweichenden, nach außen gedrängten Zughaken (21) aufnehmen kann. Die Innenkontur der Aufweitung (83) ist ein Kanal mit einer Rücksprungflanke (84), die hier eine zur Mittellinie (5) des Injektors normale Ebene darstellt. Der Übergang zwischen der beispielsweise zylindrischen Innenwandung des Auslöseelementes (82) und der Rücksprungflanke (84) ist z.B. als scharfkantige Kante (85) ausgebildet.

Zwischen dem Stempelteller (73) und dem oben liegenden Boden (39) des Gehäuses (10) sitzt vorgespannt die Schraubendruckfeder (50). Die Federkraft wird über den Stempelteller (73) auf die Zughaken (21) übertragen. Aufgrund der Neigung der Bundfläche (75) und/oder der Neigung der Abstützflächen (23) werden die Zughaken (21) keilgetriebeartig radial nach außen gedrängt. Die Auslösehülse (82) stützt diese dauernd vorhandene Radialkraft sicher ab.

Der im Gehäuse (10) angeordnete Kolbenbetätigungsstempel (60) ist hier in zwei Bereiche aufgeteilt. Der untere Bereich ist der Kolbenschieber (76). Sein Durchmesser ist etwas kleiner als der Innendurchmesser des Zylinders (101) der Zylinder-Kolben-Einheit (100). Die untere Stirnfläche des Kolbenschiebers (76) wirkt direkt auf den Kolben (111).

Der obere Bereich, der Stempelteller (73), ist eine flache, zumindest bereichsweise zylindrische Scheibe, deren Außendurchmesser einige Zehntel Millimeter kleiner ist als der Innendurchmesser des Gehäuses (10) im Mantelbereich (31).

Der Kolbenschieber (76) kann selbstverständlich auch als separates, vom Stempelteller (73) getrenntes, Bauteil ausgeführt sein. Hierzu ist er dann an der Innenwandung des Gehäuses (10) geführt.

Zwischen dem Stempelteller (73) und dem obenliegenden Boden (39) des Gehäuses (10) sitzt vorgespannt die Schraubendruckfeder (50).

Das Auslöseelement (82) liegt nach Figur 1 rückseitig an einem Gehäusebund (19) an. Auf den hinteren Abschnitt des Auslöseelements (82) und dem oberhalb des Gehäusebunds (19) liegenden Gehäusebereich ist als Sicherungselement ein in mindestens zwei Bereiche aufgeteiltes Klebeetikett (90) aufgeklebt. Der obere Bereich ist ein Abreißstreifen (91), von dem eine Abreißfahne (92) absteht. Der untere Bereich ist ein Haltestreifen (93). Der Abreißstreifen (91) und der Haltestreifen (93) haben als künftige Trennlinie eine geradlinige Perforation (94) oder eine durchgehende Materialdünnstelle. Die Perforation (94) liegt direkt über der zwischen Gehäuse (10) und Auslöseelement (82) gelegenen Montagefuge. Zum Entsichern des Injektors wird mit Hilfe der Abreißfahne (92) der Abreißstreifen (91) vom Gehäuse (10) abgezogen. Hierbei reißt der Abreißstreifen (91) an der Perforation (94) entlang dem Haltestreifen (93) ab. Es löst sich somit die Verbindung zwischen dem Gehäuse (10) und dem Auslöseelement (82).

Anstelle des Klebeetiketts mit flächiger Abreißfahne kann auch ein Klebeetikett mit eingelegtem Aufreißfaden benutzt werden. Der Aufreißfaden liegt über der Montagefuge. Als einen am Ende mehrere Millimeter überstehenden Aufreißfaden kann auch ein Kunststoffstreifen oder ein dünner Draht verwendet werden. Durch ein reißendes Abwickeln des Aufreißfadens wird das Klebeetikett im Bereich der Montagefuge in zwei Teile getrennt.

Zum Betätigen des Einweginjektors wird - nach dem Entfernen der Schutzkappe (120) - zunächst der Abreißstreifen (91) seitlich abgezogen, um das Auslöseelement (82) vom Gehäuse (10) zu lösen. Dann wird das Auslöseelement (82) mit der zur Faust geformten Hand umgriffen und der Einweginjektor auf der Injektionsstelle positioniert und gegen diese gepresst. Bei diesem Vorgang gleitet das Auslöseelement (82) auf der Außenwandung (13) des Gehäuses (10) nach vorn, also auf die Injektionsstelle zu. Die Anlageflächen (24) der Zughaken (21) rutschen über die Kante (85), vgl. Figur 2. Das Zughakenende weicht in die Aufweitung (83) aus und gibt dabei den Stempelteller (73) frei. Nun kann der Kolbenbetätigungsstempel (60) ungehindert nach unten schnellen, vgl. Figur 3. Der Zylinder (101) wird entleert.

Das Auslöseelement (82) kann selbstverständlich auch wie ein Schreibgerät gefasst werden, um es zum Betätigen gegen die Injektionsstelle zu drücken.

Figur 4 zeigt einen Injektor mit einer Auslöseeinheit (80), die das Gehäuse (10) großteils umgibt. Dazu ist am hülsenartigen Auslöseelement (82) eine Auslösekappe (59) befestigt, die den hinteren Gehäusebereich vollständig umschließt.

Des Weiteren ist am Auslöseelement (82) - anstelle des Klebeetiketts (90) - ein sichernder Auslösehebel (86) befestigt oder angeformt. Der Auslösehebel (86) hat an seinem unteren Ende ein eindrückbares Betätigungselement (81), an seinem oberen Ende eine Rastnase (87) und zwischen den Teilen (81) und (87) ein den Auslösehebel (86) lagerndes Schwenkgelenk (88). Die Rastnase (87) ragt nach Figur 4 sperrend in eine Ausnehmung (27) des Gehäuses (10) hinein.

Durch das Drücken des Betätigungselements (81) schwenkt die Rastnase (87) des Auslösehebels (86) aus der Gehäuseausnehmung (27) heraus. Die sichernde Verrastung zwischen dem Gehäuse (10) und dem hülsenartigen Auslöseelement (82) ist aufgehoben. Nun kann das Auslöseelement (82) in Richtung der Zylinder-Kolben-Einheit (100) verschoben werden.

Die Figuren 5 bis 11 zeigen eine Ausführungsform des zu Figur 4 und teilweise zu den Figuren 1 bis 3 beschriebenen Prinzips. Auch hier ist das tragende Bauteil ein einteiliges Gehäuse (10). Es wird z.B. aus einem glasfaserverstärkten Polyamid durch Spritzgießen gefertigt. Das Gehäuse (10) hat eine weitgehend rohrförmige Gestalt und ist in zwei Funktionsbereiche aufgeteilt, das ist zum einen der obere Mantelbereich (31) und zum anderen der untere Fixierbereich (41).

Der im wesentlichen rohrförmige Mantelbereich (31) ist oben durch einen z.B. ebenen Boden (39) verschlossen. In der unteren Hälfte des Mantelbereichs (31) befinden sich zwei einander gegenüberliegende, angeformte Zughaken (21). Die Anformstelle für die Zughaken (21) liegt knapp unterhalb des Bodens (39). Zur Ausbildung des jeweiligen Zughakens (21) befindet sich im Mantelabschnitt (31) ein schmaler, zumindest annähernd u-förmiger Spalt, der den einzelnen Zughaken seitlich und unten umgibt. Der Zughaken (21) hat auf ca. 80% seiner Länge die Wandstärke und die Krümmung der Wandung des Gehäuses (10). Dieser Bereich hat u.a. auch die Funktion eines federelastischen Biegebalkens (28). Er hat einen sichelförmigen Querschnitt.

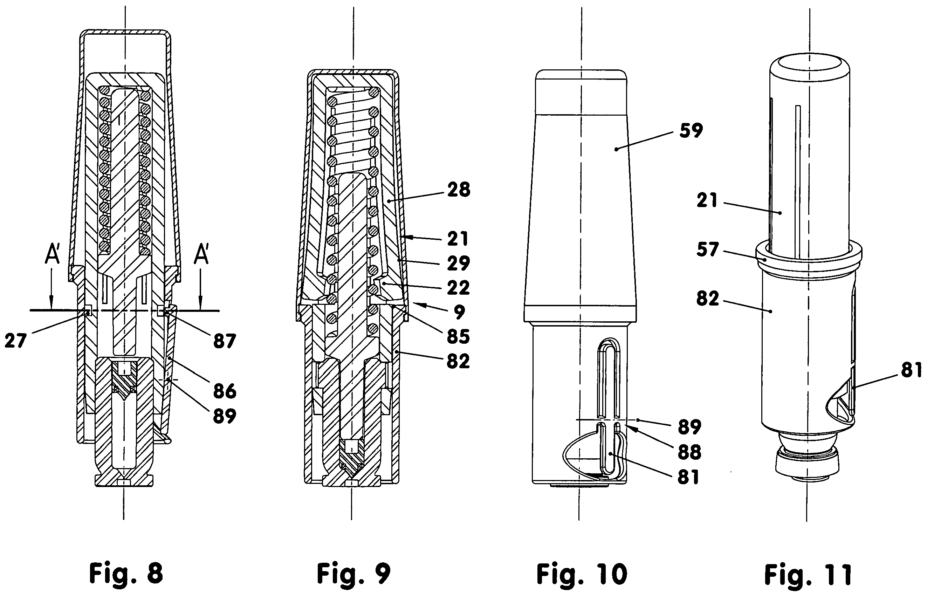

Ggf. kann ein Teil dieses Biegebalkens (28) auch mit einem rechteckigen Querschnitt ausgestattet sein, um bei der Nutzung auftretende Biegespannungen im Biegebalkenrandbereich zu reduzieren. In der Figur 9 ist der Zughaken (21) im unverformten Zustand dargestellt.

Das hier untere freie Ende des einzelnen Zughakens (21) wird durch das radial nach innen abstehenden Hakenelement (22) gebildet. Das einzelne Hakenelement (22) ragt ca. 1,5 bis 3 Millimeter nach innen über die Innenwandung des Gehäuses (10) über. Es hat zumindest eine Abstützfläche (23) und eine Anlagefläche (24). Nach Figur 5 liegt auf der Abstützfläche (23) der Stempelteller (73) des gespannten Einweg-Injektors über seine Bundfläche (75) auf. Die Abstützfläche (23), die hier die Funktion einer Keilfläche erfüllt, hat die Form eines Kegelstumpfmantels mit einem Spitzenwinkel von 120 Winkelgraden.

Ggf. haben die Zughaken (21) oder die Bundfläche (75) zumindest im Kontaktbereich eine keramische Panzerung. Es ist auch möglich, die Bundfläche (75) durch eine z.B. aufgeklebte, kegelstumpfmantelförmige und tellerfederähnliche Unterlegscheibe zu verstärken.

Die Anlageflächen (24) der Hakenelemente (22) der verformten Zughaken (21), vgl. Figur 5, entsprichen hier der Zughakenrückenfläche (29) und ist zumindest annähernd vergleichbar mit einem Teil der Außenwandung (13). Die Anlagefläche (24) kontaktiert bei gespanntem Einweg-Injektor die Innenwandung (58) des hülsenartigen Auslöseelements (82). Ggf. hat - zur Minimierung der Flächenpressung - die Anlagefläche (24) eine Krümmung, die der Innenwandung (58) entspricht.

Nach Figur 5 hat das Gehäuse (10) ca. mittig, vgl. Schnittlinie A-A, eine ringkanalartige Ausnehmung (27), in die die Rastnase (87) des Auslösehebels (86) hineingreift. Figur 6 zeigt den Eingriff im Querschnitt. In diesem Querschnitt sind auch das Auslöseelement (82) und der Kolbenschieber (76) zu erkennen.

Unterhalb des Mantelabschnitts (31) befindet sich der Fixierbereich (41) zur Aufnahme der einbaubaren Zylinder-Kolben-Einheit (100). Der Fixierbereich (41) ist Teil eines Bajonettverschlusses. Dazu sind an seiner Innenwandung zwei oder mehrere winkelförmige Kanäle (42) angeordnet, vgl. Figur 7. Die Kanäle (42) führen von der unteren Gehäusestirnseite (17) aus vertikal nach oben und gehen nach wenigen Millimetern Länge jeweils in einen kurzen horizontalen Kanalabschnitt über. Hier weist der horizontale Kanalabschnitt oberhalb des vertikal verlaufenden Kanalabschnitts eine radial durchgehende Ausnehmung auf.

Im Fixierbereich (41) ist der Zylinder (101) über z.B. zwei oder mehrere Bajonettzapfen (44) eingesetzt und fixiert, vgl. Figur 7. Ggf. befindet sich im horizontalen Kanalabschnitt oder an zumindest einem Teil der Bajonettzapfen (44) ein oder mehrere Rastelemente, die ein Lösen des Bajonettverschlusses - also ein Entfernen des Zylinders (101) - verhindern.

Der Zylinder (101) ist z.B. ein dickwandiger Topf. In der beispielsweise zylindrischen Bohrung des Zylinders (101) sitzt der stangenlose Kolben (111). Der Kolben (111) hat an seiner vorderen, zumindest annähernd kegelig gestalteten Stirnfläche eine axiale Ringnut (112) zur Aufnahme eines Dichtringes (114) oder einer dauerelastischen Dichtmasse. In der rückseitigen Stirnfläche des Kolbens (111) ist ggf. eine z.B. zylindrische Metallplatte eingelassen.

Im Zentrum der Bohrung des Zylinders (101), dessen Zylinderboden der Kontur der vorderen Kolbenstirnseite zumindest annähernd angepasst ist, befindet sich eine kurze zylindrische, düsenartige Bohrung (106). Ihr Durchmesser beträgt ca. 0,1 bis 0,5 Millimeter. Diese Bohrung (106) ist ein- bis fünfmal so lang wie ihr Durchmesser. Sie endet in einer zylindrischen Ausnehmung (107) der bodenseitigen, äußeren Stirnfläche (103) des Zylinders (101).

Im Fixierbereich (41) ist die Außenwandung des Gehäuses (10) kegelstumpfmantelförmig ausgeführt. Die Wandstärke verjüngt sich zur Stirnseite (17) hin um ca. 20%, damit das Betätigungselement (81) beim Betätigen zurückweichen kann.

Zwischen dem Kolben (111) und dem Boden (39) ist der Federenergiespeicher (50) bzw. die Antriebseinheit des Einweginjektors angeordnet. Der Federenergiespeicher (50) ist eine Schraubendruckfeder, die auf dem Kolbenbetätigungsstempel (60) mit dem Stempelteller (73) angeordnet ist. Mittels des Stempeltellers (73) stützt sich der federkraftbelastete Kolbenbetätigungsstempel (60) an den Zughaken (21) des Gehäuses (10) ab.

Der Kolbenbetätigungsstempel (60) hat oberhalb des Stempeltellers (73) einen Führungszapfen (62). Letzterer führt die Schraubendruckfeder (50). Unterhalb des Stempeltellers (73) befindet sich zentral in der Verlängerung des Führungszapfens (62) ein Kolbenschieber (76), der bei einer Betätigung des Einmalinjektors auf den Kolben (111) wirkt.

Das teilweise das Gehäuse (10) und die Zylinder-Kolben-Einheit (100) umgebende Auslöseelement (82) ist hier ebenfalls eine, z.B. aus ABS gefertigte, Auslösehülse. Die im Wesentlichen zylindrische Auslösehülse (82) hat an ihrem oberen Ende einen flanschartigen, nach außen überstehenden Kragen (57). An dem Kragen (57) ist eine Auslösekappe (59) befestigt, die das hintere Ende des Gehäuses (10) mit Spiel vollständig umgibt, vgl. auch Figur 10. Die Auslösekappe (59) hat an ihrem unteren Ende eine Ringnut (56), mit der sie auf dem Kragen (57) des Auslöseelements (82) fest sitzt.

Dieses obere Ende des Auslöseelements (82) hat als Stirnfläche die Rücksprungflanke (84) mit der innen liegenden Kante (85). Unmittelbar oberhalb der Rücksprungflanke (84) befindet sich in der Auslösekappe (59) ein als Aufweitung (83) bezeichneter Hohlraum. Oberhalb der Aufweitung (83) liegt die Auslösekappe (59) im Bereich des Bodens (39) gleitfähig an der Außenwandung (13) des Gehäuses (10) an.
Unterhalb des Kragens (57) ist im zumindest annähernd zylindrischen Bereich des Auslöseelements (82) der Auslösehebel (86) integriert, vgl. Figuren 8, 10 und 11. Letzterer ist über ein Schwenkgelenk (88), z.B. ein tordierbarer Materialsteg, mit der Auslösehülse (82) verbunden. Der Auslösehebel (86) bildet zusammen mit dem Betätigungselement (81) eine Wippe, die im Bereich des Schwenkgelenks (88) ihre Schwenkachse (89) hat, vgl. auch Figur 10. Wird der Auslösehebel (86) durch ein Drücken auf das Betätigungselement (81) gegen das Gehäuse (10) gepresst, verlässt am anderen Ende des Auslösehebels (86) die Rastnase (87) entsichernd die Ausnehmung (27), vgl. Figur 8. Zum Schnittverlauf A'-A' gibt es keine Darstellung, er entspricht jedoch dem Schnittverlauf A-A.

Die Figur 8 zeigt den Einweginjektor mit betätigtem Auslösehebel, also entsichert. In Figur 9 ist der Injektor mit nach unten geschobenem Auslöseelement (82) dargestellt.

Mit dem nach unten Schieben der Auslösehülse (82) rutschen die Biegebalken (28), vgl. Figur 9, mit ihrer Rückenfläche (29) über die Kante (85) nach außen in die Aufweitung (83). Die Zughaken (21) biegen sich elastisch nach außen in ihre eigentliche Ausgangslage. Die nun nicht mehr verformten Zughaken (21) geben den Kolbenbetätigungsstempel (60) frei, so dass der Kolben (111) unter der Wirkung des Federelements (50) ruckartig in den Zylinder (101) eingeschoben wird. Mit der Abgabe des Medikaments über die Zylinder-Kolben-Einheit (100) ist der Injektionsvorgang beendet.

Bei dieser Ausführungsvariante sind mit Ausnahme des Federelements (50) alle Bauteile rotationssymmetrisch und/oder zu einer auf der Mittellinie (5) gelegenen Ebene spiegelsymmetrisch aufgebaut, was die Montage vereinfacht.

Bei Injektoren, bei denen der Kolbenbetätigungsstempel (60) im Gehäuse (10) - zumindest abschnittsweise - mit geringem Spiel geradgeführt ist und der Kolbenbetätigungsstempel (60) eine ausreichende Biegefestigkeit aufweist, kann anstatt zwei oder mehrerer Zughaken (21) auch nur ein einziger Zughaken (21) verwendet werden.

Bei den in den Figuren dargestellten Varianten ist die einzelne Kontaktzone zwischen dem Zughaken (21) und dem Stempelteller (73), als Flächen (23) und (75) ausgeführt, die gleitfähig einander kontaktieren. In einer besonderen Ausgestaltung kann in jeder Fläche (23) der einzelnen Zughaken (21) eine Walze gelagert werden, die bei einer Betätigung des Injektors an der Fläche (75) des Stempeltellers wälzgelagert, also reibungsarm, abrollt.

Mit Ausnahme des Federelements (50), einer ggf. vorhandenen Kolbenplatte und der beispielsweise vorhandenen Lagerwalzen der Zughaken (21) sind alle Teile der zuvor beschriebenen Einweg-Injektoren aus Kunststoffen oder kunststoff- bzw. gummiähnlichen Werkstoffen gefertigt.

### Bezugszeichenliste:

- 1: Injektionslösung; Medikament
- 5: Mittellinie des Einweginjektors
- 6: Auslösebewegungsrichtung von (82), Abwärtsbewegung
- 8: Sperrstellung
- 9: Lösestellung

- 10: Gehäuse, einteilig
- 13: Außenfläche, zylindrisch
- 16: Auslösebereich, oben
- 17: untere Gehäusestirnseite
- 19: Gehäusebund

- 21: Zughaken
- 22: Hakenelemente
- 23: Abstützfläche, Abstützkante
- 24: Anlagefläche
- 27: Ausnehmung, Ringnut
- 28: Biegebalken, biegeelastisches Element
- 29: Rückenfläche

- 31: Mantelbereich
- 33: Durchbrüche
- 39: Boden

- 41: Fixierbereich für die Zylinder-Kolben-Einheit
- 42: Kanäle, winkelförmig
- 44: Bajonettzapfen

- 50: Federelement, Schraubendruckfeder, Federenergiespeicher
- 52: Federelement von (21)

- 56: Ringnut an (59)
- 57: Kragen an (82)
- 58: Innenwandung von (82)
- 59: Auslösekappe

- 60: Kolbenbetätigungsstempel
- 62: Führungszapfen

- 73: Stempelteller
- 75: Bundfläche, Stirnfläche, unten
- 76: Kolbenschieber

- 80: Auslöseeinheit
- 81: Betätigungselement
- 82: Auslöseelement
- 83: Aufweitung
- 84: Rücksprungflanke
- 85: Kante, scharfkantig
- 86: Auslösehebel, Sicherungselement
- 87: Rastnase
- 88: Schwenkgelenk
- 89: Schwenkachse

- 90: Klebeetikett, Sicherungselement
- 91: Abreißstreifen
- 92: Abreißfahne
- 93: Haltestreifen
- 94: Sollbruchstelle, Perforation
- 100: Zylinder-Kolben-Einheit
- 101: Zylinder
- 103: Stirnfläche
- 106: Bohrung, Düse
- 107: Ausnehmung in der Stirnfläche
- 111: Kolben
- 112: Ringnut
- 114: Dichtring, Dichtung

- 120: Schutzkappe, Klebeversiegelung

## Patentansprüche

1. Einweginjektor mit einem Gehäuse (10), in dem oder an dem - jeweils zumindest bereichsweise - mindestens ein mechanischer Federenergiespeicher, mindestens eine - zumindest zeitweise wirkstoffbefüllbare - Zylinder-Kolben-Einheit (100), mindestens ein Kolbenbetätigungsstempel (60) und mindestens eine Auslöseeinheit (80) angeordnet ist,
- wobei der Federenergiespeicher (50) mindestens ein vorgespanntes Federelement umfasst und
- wobei zumindest ein Teil des Kolbenbetätigungsstempels (60) zwischen dem Federenergiespeicher (50) und einem Kolben (111) der Zylinder-Kolben-Einheit (100) positioniert ist,
- wobei das Gehäuse (10) mindestens einen Zughaken (21) aufweist,
- wobei der einzelne Zughaken (21) jeweils im Bereich seines freien Endes mindestens eine Abstützfläche (23) hat,
- wobei an der Abstützfläche (23) der federbelastete Kolbenbetätigungsstempel (60) gegen eine Auslösung des Einweginjektors sperrende Lage anliegt,
- wobei die zwischen dem Zughaken (21) und dem Kolbenbetätigungsstempel (60) gelegene Kontaktzone ein den Zughaken (21) radial nach außen drängendes Keilgetriebepaar darstellt,
- wobei die sperrende Lage des Zughakens (21) durch ein in einer Sperrstellung (8) positioniertes Auslöseelement (82) gesichert ist und
- wobei das Auslöseelement (82) eine Lösestellung (9) hat, die ein radial nach außen gerichtetes Zurückweichen des Zughakens (21) - unter Freigabe des Kolbenbetätigungsstempels (60) - bewirkt,
**dadurch gekennzeichnet, dass**
- der oder die Zughaken (21) zumindest bereichsweise als biegeelastische - nach außen federnde - Elemente (28), die einen Biegebalken darstellen, am Gehäuse (10) angeformt sind,
- das Auslöseelement (82) an seinem oberen Ende einen flanschartigen, nach außen überstehenden Kragen (57) aufweist, an dem eine Auslösekappe (59) befestigt ist, die das hintere Ende des Gehäuses (10) mit Spiel vollständig umgibt und
- die Auslösekappe (59) eine Aufweitung (83) aufweist die so ausgebildet ist, dass mit dem nach unten in Richtung der Zylinder-Kolben-Einheit Schieben der Auslösehülse (82) in die Lösstellung die Biegebalken (28) nach außen in die Aufweitung (83) rutschen und in der Aufweitung (83) aufgenommen sind.

2. Einweginjektor gemäß Anspruch 1,
**dadurch gekennzeichnet,**
**dass** jeder Zughaken (21) eine Rückenfläche (29) hat, die in der Sperrstellung (8) Teil der zylindrischen Außenfläche (13) des Gehäuses (10) sind.

3. Einweginjektor gemäß Anspruch 1,
**dadurch gekennzeichnet,**
**dass** die Abstützfläche (23) des oder der Zughaken (21) eine Innenfläche eines Torusmantelteils oder einer Kegelstumpfmantelfläche ist.

4. Einweginjektor gemäß Anspruch 3,
**dadurch gekennzeichnet,**
**dass** bei einer bereichsweise torusmantelförmigen Abstützfläche (23) die Abstützfläche (23) in einer Ebene konvex und zugleich in einer anderen Ebene konkav gekrümmt ist.

5. Einweginjektor gemäß Anspruch 1,
**dadurch gekennzeichnet,**
**dass** das Auslöseelement (82) eine auf dem Gehäuse (10) gleitgelagerte Hülse ist, an der - in der Sperrstellung (8) - der oder die Zughaken (21) mit ihren Anlageflächen (24) radial anliegen.

6. Einweginjektor gemäß Anspruch 1,
**dadurch gekennzeichnet,**
**dass** am Auslöseelement (82) ein schwenkbarer Auslösehebel (86) gelagert ist, der eine Rastnase (87) hat, die sichernd in eine Ausnehmung (27) des Gehäuses (10) eingreift.

7. Einweginjektor gemäß Anspruch 1,
**dadurch gekennzeichnet,**
**dass** das Auslöseelement (82) in Kombination mit dem Gehäuse (10) und einer an ihm befestigten Abreißbanderole (90) eine Auslöseeinheit (80) bildet.

## Claims

1. Disposable injector with a housing (10), in which or on which - respectively at least in certain areas - at least one mechanical spring-energy storage, at least one cylinder-piston unit (100) which can be filled at least occasionally with active ingredient, at least one piston-actuating plunger (60) and at least one actuating unit (80) are arranged,
- whereby the spring-energy storage (50) includes at least one pre-stressed spring-loaded element and
- whereby at least part of the piston-actuating plunger (60) is positioned between the spring-energy storage (50) and a piston (111) of the cylinder-piston unit (100),
- whereby the housing (10) has at least one draw hook (21),
- whereby the individual draw hook (21) has at least one support surface (23) respectively in the region of its free end,
- whereby the spring-loaded piston-actuating plunger (60) rests on the support surface (23) against a position which prevents any actuation of the disposable injector,
- whereby the contact zone placed between the draw hook (21) and the piston-actuating plunger (60) represents a variable-speed gear pair thrusting the draw hook (21) radially outwards,
- whereby the locking position of the draw hook (21) is secured by an actuating element (82) positioned in a locked position (8), and
- whereby the actuating element (82) has a triggering position (9) which effects radially outwards aligned retreat of the draw hook (21) when the piston-actuating plunger (60) is released,
**characterized in that**
- the draw hook or the draw hooks (21) is/are formed at least in certain areas as flexural elastic-outward-springing-elements (28) on the housing (10), which elements represent a flexional beam,
- the release element (82) has at its upper end a flange-like, outwardly protruding collar (57) to which an actuating cap (59) is attached, which fully encloses the rear end of the housing (10) with clearance, and
- the actuating cap (59) has a widened region (83) which is configured such that, when the release sleeve (82) is pushed down into the triggering position in the direction of the cylinder-piston unit, the flexional beams (28) slip outwards into the widened region (83) and are taken up in the widened region (83).

2. Disposable injector according to Claim 1, **characterized in that** each draw hook (21) has a rear surface (29) which is part of the cylindrical outer surface (13) of the housing (10) in the locked position (8) .

3. Disposable injector according to Claim 1, **characterized in that** the support surface (23) of the draw hook or of the draw hooks (21) is an inner surface of a toroid jacket part or a frustoconical surface.

4. Disposable injector according to Claim 3, **characterized in that** the support surface (23) is curved convexly in one plane and at the same time is curved concavely in another plane in the case of a support surface (23) toroid-jacket-shaped in certain areas.

5. Disposable injector according to Claim 1, **characterized in that** the actuating element (82) is a sleeve slidably mounted on the housing (10), on which the draw hook or the draw hooks (21) abut radially by their contact surfaces (24), in the locked position (8) .

6. Disposable injector according to Claim 1, **characterized in that** mounted on the actuating element (82) is a pivotable release lever (86) which has a latching tab (87) engaging securely in a recess (27) of the housing (10).

7. Disposable injector according to Claim 1, **characterized in that**, in combination with the housing (10) and a tear-off banderole (90) fastened thereon, the actuating element (82) forms an actuating unit (80) .

## Revendications

1. Injecteur à usage unique, comprenant un boîtier (10) dans lequel ou sur lequel sont disposés à chaque fois au moins en partie au moins un accumulateur d'énergie mécanique à ressort, au moins une unité cylindre-piston (100) pouvant être remplie au moins temporairement d'une substance active, au moins un poinçon d'actionnement de piston (60) et au moins une unité de déclenchement (80),
- l'accumulateur d'énergie à ressort (50) comprenant au moins un élément de ressort précontraint et
- au moins une partie du poinçon d'actionnement de piston (60) étant positionnée entre l'accumulateur d'énergie à ressort (50) et un piston (111) de l'unité cylindre-piston (100),
- le boîtier (10) présentant au moins un crochet de traction (21),
- le crochet de traction unique (21) présentant à chaque fois dans la région de son extrémité libre au moins une surface de support (23),
- le poinçon d'actionnement de piston sollicité par ressort (60) s'appliquant au niveau de la surface de support (23) contre une position bloquant déclenchement de l'injecteur à usage unique,
- la zone de contact située entre le crochet de traction (21) et le poinçon d'actionnement de piston (60) constituant une paire d'organes de calage repoussant radialement vers l'extérieur le crochet de traction (21),
- la position de blocage du crochet de traction (21) étant fixée par un élément de déclenchement (82) positionné dans une position de blocage (8) et
- l'élément de déclenchement (82) présentant une position de libération (9) qui provoque un retour du crochet de traction (21) orienté radialement vers l'extérieur, en libérant le poinçon d'actionnement de piston (60), **caractérisé en ce que**
- le ou les crochets de traction (21) sont formés sur le boîtier (10) au moins en partie sous forme d'éléments flexibles élastiques (28), à effet de ressort vers l'extérieur, qui constituent une poutre flexible,
- l'élément de déclenchement (82) présente, au niveau de son extrémité supérieure, un collet en forme de bride (57) dépassant vers l'extérieur, sur lequel est fixé un capuchon de déclenchement (59) qui entoure complètement avec jeu l'extrémité arrière du boîtier (10), et
- le capuchon de déclenchement (59) présente un élargissement (83) qui est réalisé de telle sorte qu'avec le déplacement de la douille de déclenchement (82) vers le bas dans la direction de l'unité cylindre-piston, dans la position de libération, les poutres flexibles (28) glissent vers l'extérieur dans l'élargissement (83) et soient reçues dans l'élargissement (83).

2. Injecteur à usage unique selon la revendication 1, **caractérisé en ce que**
chaque crochet de traction (21) présente une surface arrière (29), lesquelles, dans la position de blocage (8), font partie de la surface extérieure cylindrique (13) du boîtier (10).

3. Injecteur à usage unique selon la revendication 1, **caractérisé en ce que**
la surface de support (23) du ou des crochets de traction (21) est une surface intérieure d'une partie d'enveloppe en forme de tore ou d'une surface d'enveloppe tronconique.

4. Injecteur à usage unique selon la revendication 3, **caractérisé en ce que**
dans le cas d'une surface de support (23) en partie en forme d'enveloppe de tore, la surface de support (23) est courbée de manière convexe dans un plan et de manière concave simultanément dans un autre plan.

5. Injecteur à usage unique selon la revendication 1, **caractérisé en ce que**
l'élément de déclenchement (82) est une douille supportée de manière coulissante sur le boîtier (10), contre laquelle s'appliquent radialement dans la position de blocage (8) le ou les crochets de traction (21) avec leur surface d'appui (24).

6. Injecteur à usage unique selon la revendication 1, **caractérisé en ce que**
un levier de déclenchement pivotant (86) est supporté sur l'élément de déclenchement (82), lequel présente un ergot d'encliquetage (87) qui s'engage en réalisant une fixation dans un évidement (27) du boîtier (10).

7. Injecteur à usage unique selon la revendication 1, **caractérisé en ce que**
l'élément de déclenchement (82) conjointement avec le boîtier (10) et une banderole de déchirure fixée sur celui-ci (90) forme une unité de déclenchement (80).
